# FASCICULE DE BREVET EUROPEEN

(11) **EP 2 999 440 B1**
(45) Date de publication et mention de la délivrance du brevet: **25.08.2021**
(21) Numéro de dépôt: 14731706.9
(22) Date de dépôt: 22.05.2014
(51) Int. Cl.: A61F 5/01, A47C 31/12, A47C 7/40, A47C 7/44, A47C 7/46, A61G 5/10, A61G 5/12

(54) **DISPOSITIF MODULAIRE DE PROTECTION DORSALE**
MODULARE VORRICHTUNG FÜR DORSALEN SCHUTZ
MODULAR DEVICE FOR DORSAL PROTECTION

(30) Priorité: 23.05.2013 FR 1354649
(43) Date de publication de la demande: 30.03.2016
(73) Titulaire: Evom, 34980 St Clément de Rivière (FR)
(72) Inventeur: DESVAUX DE MARIGNY, Christopher, 34980 St Clément de Rivière (FR)
(74) Mandataire: Rhein, Alain
(86) Numéro de dépôt international: PCT/FR2014/051204
(87) Numéro de publication internationale: WO 2014/188132

(56) Documents cités:
- WO-A1-02/22067
- WO-A1-2012/149978
- WO-A1-2013/174484
- US-A- 4 658 807
- US-A1- 2006 181 126
- US-A1- 2012 157 901
- None

## Description

La présente invention entre dans le domaine des protections dorsales.

L'invention concerne un dispositif modulaire de protection dorsale articulée, suivant la revendication 1 et un fauteuil roulant comprenant le dispositif modulaire de protection suivant la revendication 9.

Un tel dispositif trouvera une application préférentielle, mais aucunement limitative, dans le domaine médical et paramédical, sous la forme d'une coque protectrice externe, destinée à être apposée et maintenue dans le dos d'un patient, à la manière d'une orthèse, mais prévue articulée pour autoriser mais surtout améliorer les mouvements dudit patient.

On notera qu'un tel dispositif de protection pourra servir à appareiller une personne à mobilité réduite, formant un dossier articulé personnel à son utilisateur, susceptible de venir en remplacement ou en complément du dossier de son fauteuil roulant.

De manière connue, une personne à mobilité réduite dont les déplacements nécessitent un fauteuil roulant, se retrouve confrontée à la rigidité du dossier contre lequel elle reste en appui. Actuellement, les dossiers équipant les fauteuils roulants sont droits et rectilignes, complètement inadaptés à la morphologie du dos présentant une forme globalement en S, pourvue de ses cyphoses et ses lordoses. Par conséquent, l'appui prolongé contre un dossier droit et statique engendre des discopathies douloureuses. De plus, cette rigidité entraine une certaine immobilité du patient, empêchant ou limitant les mouvements dorsaux nécessaires aux disques intervertébraux pour conserver leur souplesse et, ainsi, continuer à jouer leur rôle d'amortisseurs.

D'autre part, une paralysie, dont est atteinte une personne à mobilité réduite, entraine une atrophie des muscles. Dès lors, le manque d'effort et d'activité physique provoque un manque d'oxygénation des cellules du corps. Dans ces conditions, l'appui contre le dossier ne s'effectue pas uniformément au niveau de la totalité de la surface du dos du patient, mais sur des points d'appui limités situés au niveau des épineuses de la colonne vertébrale, devenues davantage saillantes.

Un autre inconvénient réside dans l'impact de l'inertie prolongée de l'appui en contact quasi-permanent au niveau de la peau du dos. Le derme et l'épiderme se retrouvent alors comprimés. Le contact empêche la peau de respirer convenablement, provoquant des engourdissements allant jusqu'à l'ischémie des tissus et le risque d'apparition d'escarre.

Par ailleurs, pour compenser la perte musculaire, une personne à mobilité réduite doit porter une ceinture abdominale afin de maintenir les viscères sur tout le tour de son ventre, directement au contact de la peau. Une telle ceinture frotte et forme des plis, rendant contraignant et inconfortable son port. De plus, l'appui lié aux plis au niveau du bas du dos, peut provoquer à la longue un changement néfaste des positions des lombaires de la colonne vertébrale.

Afin de diminuer les inconvénients dus au positionnement prolongé, une cale dorsale peut être disposée entre le dos et le dossier du fauteuil, au niveau lombaire. Toutefois, cette cale, s'apparentant davantage à un coussin, n'est ni reliée au siège ni à la personne, de sorte qu'elle se déplace et ne garde pas sa position, générant un inconfort.

Pour tenter de pallier ces inconvénients, une solution existante consiste en des fauteuils roulants équipés d'une assise et d'un dossier ergonomiques, pourvus d'une couche matelassée. Ces équipements sont souvent plus lourds et coûteux, améliorant uniquement le confort, sans réellement traiter les contraintes fondamentales liées à la stature dorsale du corps humain.

Il existe aussi des fauteuils dont le dossier possède en partie basse une articulation en charnière, autorisant leur rotation depuis une position verticale vers des positions inclinées vers l'arrière, et inversement. Ce changement de position nécessite une manipulation contraignante, voire fastidieuse, de la personne sur un organe de manœuvre. De plus, les positions inclinées ne confèrent pas une assise confortable, permettant juste à la personne d'appliquer un effet de détente provisoire de son dos pour soulager les tensions sur les disques intervertébraux.

Une solution alternative consiste en une coque dorsale, s'apparentant à un corset médical. Un exemple d'une telle coque est décrit au travers du document WO 96/07344. Elle est constituée d'une armature rigide sur laquelle sont fixées latéralement des lames incurvées en matériau flexible. Des moyens adaptés assurent la déformation des lames par appui extérieur depuis ladite armature fixe et rigide. Ainsi, il est possible d'ajuster la forme desdites lames en fonction de la morphologie dorsale du patient. De plus, un panneau vertical central présente un bombage reprenant la courbure lombaire du dos. Une telle coque assure donc un maintien du dos, avec un réglage très précis. Toutefois, une fois en place, ce corset constitue une unique coque rigide, n'autorisant aucun mouvement du dos. De plus, la forme plate du panneau vertical pose encore les inconvénients de l'appui sur les pointes des épineuses et de la compression de la peau.

Un autre exemple de coque est décrit dans le document EP 0 109 572. Il comprend encore une fois une armature fixe recevant en fixation des plaques en matériau déformable, de manière à être pliées pour venir se conformer à la morphologie anatomique de la personne. Le but de cette coque est de fixer et maintenir certaines parties du corps d'un patient, notamment en vue de son rétablissement ou de sa rééducation. Encore une fois, cette coque s'apparente à un corset qui, une fois positionné, n'autorise aucune liberté de mouvement à son porteur, générant davantage d'inconvénients que le dossier droit d'un fauteuil roulant.

On connait par ailleurs par le document WO02/22067 dossier, notamment de fauteuil roulant, comprendre une structure articulée susceptible de se déformer en flexion et en torsion intégrant des moyens de rappel élastique aménagé au niveau de chaque articulation de la structure. Les moyens de rappel élastique comportent au moins un ressort et notamment des ressorts de torsion et des ressorts de flexion.

Plus précisément, la structure articulée est composée d'une succession d'articulations à pivotement autour d'un axe sensiblement horizontal intégrant des ressorts de rappel autour de cet axe et entre lesquelles sont intercalées des articulations à axe de pivotement vertical équipées elles aussi de ressort de rappel.

Sur les articulation à pivotement horizontal sont montées des lames de maintien latérales. Ce document (WO 02/22067 A1) décrit le préambule de la revendication 1.

La présente invention a pour but de pallier les inconvénients de l'état de la technique, en proposant un dispositif modulaire de protection dorsale articulée.

Pour ce faire, tout d'abord, un tel dispositif est prévu modulaire, pourvus de plusieurs éléments de maintien entièrement dissociés les uns des autres. En fonction du nombre d'éléments, un maintien peut être assuré depuis le niveau lombaire en bas du dos jusqu'au niveau dorsal, voir cervical. Préférentiellement, ledit dispositif comprend au moins deux éléments de maintien. Ainsi, il est possible d'adapter le nombre d'éléments en fonction de la personne qui va le porter et de son handicap.

Par ailleurs, chaque élément de maintien présente une forme ergonomique globalement incurvée en arc de cercle, reprenant la forme arrondie du dos selon sa largeur. A ce titre, en fonction de la position au niveau de laquelle chaque élément vient en appui contre le dos, chaque élément présente alors des dimensions différentes, plus large aux niveaux lombaire, thoracique et dorsal supérieur et moins large au niveau dorsal inférieur. En outre, la forme en arc de cercle peut présenter, au niveau central, un renfoncement prévu pour recevoir la saillie des épineuses de la colonne vertébrale, assurant un appui essentiellement de part et d'autre de ces dernières. Ainsi, cette forme anthropomorphique vient parfaitement s'adapter au dos de la personne.

De plus, chaque élément peut être constitué d'un matériau rigide ou semi-rigide, autorisant uniquement une déformation limitée par flexion ou écartement des extrémités de ses éléments de maintien. Ainsi, ces derniers viennent bien en appui et maintenir indépendamment chaque partie du dos.

En outre, il est possible d'appliquer directement le dispositif contre la peau du dos, sous les vêtements, servant d'interface avec le dossier contre lequel la personne s'appui, mais aussi avec le tissu, évitant de provoquer des plis et des frottements.

D'autre part, une caractéristique essentielle de ce dispositif réside dans l'articulation de ses éléments de maintien. En effet, l'invention prévoit d'autoriser le déplacement des éléments de maintien les uns par rapport aux autres selon au moins deux degrés de liberté de mouvement, en torsion et en flexion. Ainsi, le porteur peut tourner uniquement une partie de son dos, les épaules par rapport au bassin, tout en conservant la protection en place contre le dos. Il peut aussi s'étendre en arquant le dos, en faisant corps avec le dispositif.

De plus, les articulations entre chaque élément sont prévues élastiques, de manière à autoriser leur déformation mais à appliquer une force de rappel élastique vers la position d'origine. Ainsi, les mouvements du porteur reçoivent une contrainte, nécessitant un effort qui entretient et assure le développement musculaire, tout en favorisant les déplacements d'une partie du dos et, de ce fait, l'entretien de l'élasticité des disques intervertébraux.

Par ailleurs, lesdites articulations permettent de ménager un espace entre les éléments de maintien alors séparés, empêchant qu'ils ne se touchent ou ne frottent l'un contre l'autre lors de leurs déplacements respectifs.

Dès lors, le dispositif de coque selon l'invention constitue une protection articulée propre à chaque personne et configurée pour la morphologie de son dos, faisant corps à la manière d'une orthèse.

De façon subsidiaire, cette protection dorsale peut constituer un dossier, lorsqu'elle est équipée, en partie inférieure, de moyens de fixation amovible avec ledit fauteuil. Dès lors, la personne peut venir assujettir à sa guise sa protection dorsale avec son fauteuil, ou l'en détacher, offrant une liberté de mouvement inégalée actuellement pour une personne à mobilité réduite.

En outre, cette protection dorsale pouvant être appliquée directement contre la peau, il est possible de la porter sous ses vêtements.

Ainsi l'invention concerne un dispositif modulaire de protection dorsale suivant la revendication 1.

Selon le mode préférentiel de réalisation, ladite pièce peut être constituée en polyuréthane.

Avantageusement, ledit dispositif peut comprendre au moins un troisième élément relié supérieurement audit second élément par l'intermédiaire d'une liaison articulée identique.

En particulier, un tel dispositif peut encore comprendre au moins un quatrième élément relié supérieurement audit troisième élément par l'intermédiaire d'une liaison articulée identique.

Par ailleurs, chaque élément peut être constitué d'une unique lame incurvée en face avant.

Selon une autre caractéristique subsidiaire, ladite liaison peut constituer des moyens de séparation espacée desdits éléments.

L'invention concerne encore un fauteuil roulant comprenant le dispositif modulaire de protection suivant la revendication 9.

D'autres caractéristiques et avantages de l'invention ressortiront de la description détaillée qui va suivre des modes de réalisation non limitatifs de l'invention, en référence aux figures annexées, dans lesquelles :
- les figures 1 à 3 représentent selon une vue en perspective de trois quart, trois modes de réalisations différents dudit dispositif, dans lesquels ce dernier est pourvu, respectivement, de deux, trois et quatre éléments de maintien ;
- la figure 4 représente schématiquement le mode de réalisation du dispositif de la figure 2 selon une vue de dessus ;
- la figure 5 représente schématiquement en perspective le mode de réalisation de la figure 4 en vue éclatée, montrant les différentes parties le constituant et les fixations entre elles ; et
- la figure 6 représente schématiquement le mode de réalisation de la figure 4 selon une vue de derrière.

La présente invention concerne un dispositif 1 modulaire de protection dorsale articulée.

Un tel dispositif 1 s'apparente à une coque conçue en plusieurs parties assujetties les unes par rapport aux autres pour autoriser leurs déplacements relatifs, tout en assurant le maintien au contact du dos de son porteur. En somme, les différentes parties présentent une certaine mobilité et peuvent se déplacer l'une par rapport à l'autre sous l'action des mouvements de l'utilisateur, en particulier des mouvements des différentes parties de son dos, comme tourner les épaules ou le haut du buste par rapport au bassin, se cambrer en arrière ou se pencher vers l'avant.

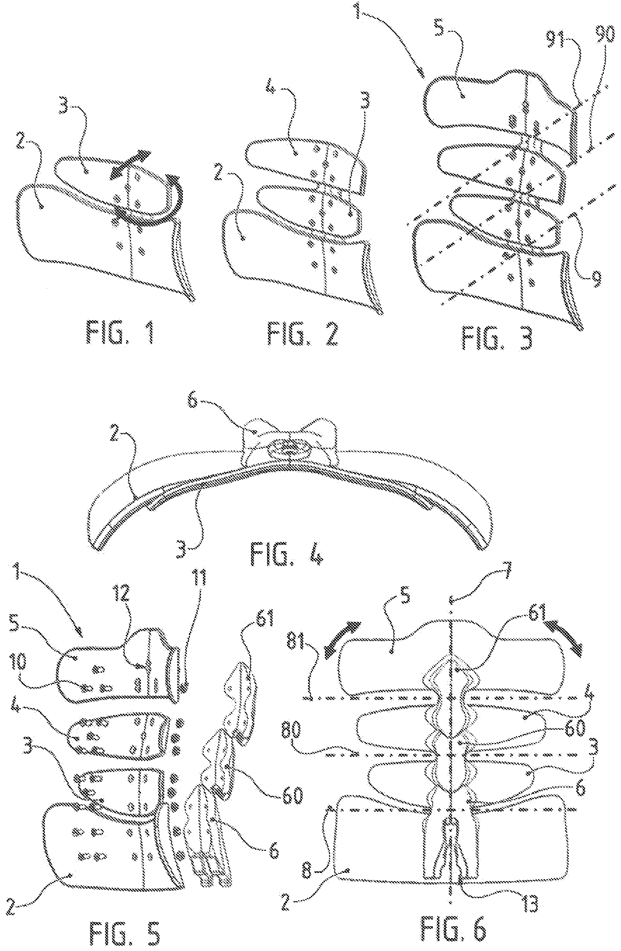

Pour ce faire, ledit dispositif comprend au moins un premier élément inférieur 2 et un second élément supérieur 3 de maintien et d'appui dorsal. En fonction du dos de son porteur, mais aussi de son handicap éventuel, ledit dispositif 1 peut comprendre davantage d'éléments de maintien 2, 3, à savoir au moins deux, trois ou quatre, voire encore davantage. La figure 1 montre un dispositif 1 pourvu de deux éléments de maintien 2, 3, tandis que sur la figure 2, il comprend un troisième élément de maintien 4, et que sur la figure 3, cette coque est constituée d'un quatrième élément de maintien 5.

On notera que le premier élément 2 est toujours situé en partie inférieure du dispositif 1, destiné à être appliqué au niveau du bas du dos, en vis-à-vis des lombaires, tandis que le deuxième élément 3 vient se positionner juste au-dessus, appliqué au milieu du dos, comme le troisième élément 4 venant encore au-dessus, dans le haut du dos, en vis-à-vis des vertèbres thoraciques.

En outre, un élément supplémentaire, non représenté, peut être positionné encore au-dessus, au niveau des cervicales.

En outre, ledit dispositif 1 se veut ergonomique et anthropomorphique. Pour ce faire, chaque élément 2, 3, 4, 5 est constitué d'une unique lame incurvée en face avant. En somme, une telle lame présente une courbure selon un arc de cercle s'étendant de manière à ménager une concavité arrondie en face avant, destinée à recevoir le dos de son porteur.

De plus, cette concavité peut comprendre, au niveau central, un creux formé par une discontinuité dans la régularité de la courbure de la lame. Ce creux est orienté vers l'arrière, formant de part et d'autre des bossages. Ledit creux est destiné à recevoir les épineuses de la colonne vertébrale, tandis que lesdits bossages viennent en appui de part et d'autre de cette dernière.

Cette configuration, particulièrement visible sur la figure 4, permet ainsi d'améliorer le confort et l'appui du dos contre et au sein dudit dispositif 1.

En outre, chaque lame constituant un élément peut présenter des dimensions en longueur, mais aussi en hauteur, ainsi qu'une courbure, adaptées à la portion du dos contre laquelle elle est destinée à venir en appui.

De façon essentielle, deux éléments sont reliés entre eux en faces arrières par l'intermédiaire d'au moins une liaison articulée 6. Ainsi, dans le cas de deux éléments 2 et 3, ils sont reliés par une unique liaison 6. Dans le cas de trois éléments 2, 3 et 4, ils sont reliés deux à deux par deux liaisons 6 et 60. Dans le cas de quatre éléments, 2, 3, 4 et 5, ils sont reliés deux à deux par trois liaisons 6, 60 et 61.

En particulier, comme visible sur les figures 5 et 6, la liaison 60 (ou 61) d'un couple d'éléments supérieurs 3 et 4 (ou respectivement 5 et 6) peut venir recouvrir au moins partiellement, par l'arrière, la liaison 6 (ou respectivement 60) d'un couple d'éléments 2 et 3 (ou respectivement 3 et 4) situé en dessous.

Selon une caractéristique préférentielle, chaque liaison 6, 60, 61 constitue des moyens de séparation espacée desdits éléments. En somme, elle relie sa paire d'éléments 2 et 3, 3 et 4, 4 et 5, de manière à ménager un espace entre eux, pour autoriser leur déplacement relatif sans risque que lesdits éléments ne se touchent ou ne frottent, notamment au niveau de leurs bords respectifs.

Selon une caractéristique essentielle, ladite liaison articulée 6, 60, 61 possède au moins deux degrés de liberté en torsion et en flexion. En d'autres terme, chaque liaison 6, 60, 61 autorise la rotation de deux éléments entre eux selon, d'une part, autour d'un premier axe virtuel 7 passant par ladite liaison 6, 60, 61 et s'étendant sensiblement verticalement et, d'autre part, autour d'un second axe virtuel 8 passant approximativement par le centre de ladite liaison 6, 60, 61 et s'étendant sensiblement horizontalement et approximativement dans le plan d'appui dorsal, ou parallèlement à ce dernier.

On notera que ledit premier axe 7 est identique pour toutes les liaisons 6, 60, 61, alors que chaque 6, 60, 61 possède un second axe 8, 80, 81 indépendant qui lui est propre.

Ces deux rotations sont modélisées sur la figure 1, tandis que la figure 6 fait apparaître lesdits axes 6.

En somme, la rotation autour du premier axe 7 autorise la rotation de chaque élément lorsque le porteur tourne une partie de son dos, comme les épaules ou le buste par rapport au bassin. De plus, la rotation autour de l'une et/ou l'autre desdits seconds axes 8, 80, 81 permet au porteur de se cambrer vers l'arrière ou de se pencher vers l'avant.

Selon un mode particulier de réalisation, ladite liaison articulée 6, 60, 61 peut posséder au moins un troisième degré de liberté en flexion. Ce troisième degré se traduit par une rotation autour d'un troisième axe virtuel 9, 90, 91 s'étendant approximativement orthogonalement aux plans contenant lesdits seconds axes 8, 80, 81.

Une telle rotation est représentée sur la figure 6 pour le quatrième élément 5 et correspond à un mouvement du haut du buste lorsque le porteur se penche vers son côté droit ou son côté gauche. Un tel mouvement peut s'appliquer au niveau des liaisons 6, 60, 61 de chaque couple d'éléments, possédant chacun un troisième axe 9, 90, 91.

Ainsi, chaque liaison 6, 60, 61 s'apparente donc à une articulation multiaxes.

Selon une caractéristique additionnelle, ladite liaison 6, 60, 61 peut être prévue élastique. En d'autres termes, elle applique une force de rappel élastique vers la position d'origine lors des différentes rotations et des déplacements des éléments entre eux, induits par les mouvements du porteur. Ladite position d'origine correspond au dispositif 1 tel que visible sur les figures, lorsqu'il est placé en soutien et maintien dorsal de son porteur, sans contrainte sur les liaisons 6, 60, 61, afin que le dos dudit porteur soit maintenu dans une position naturelle.

De plus, le caractère élastique de chaque liaison 6, 60, 61 peut être obtenu au travers du matériau la constituant. Pour ce faire, ladite liaison 6, 60, 61 est constituée par une unique pièce de jonction en matériau élastique de type élastomère. Un tel matériau assure la déformation de ladite pièce, puis son retour à la forme d'origine.

Selon le mode préférentiel de réalisation, ladite pièce peut être constituée en polyuréthane. Ce matériau possède les caractéristiques d'élasticité requises, pour une résistance accrue à la déformation, ainsi qu'une bonne tenue dans le temps et une usure moindre.

Comme visible sur les figures 5 et 6, chaque pièce peut présenter une forme spécifique allongée depuis une extrémité inférieure jusqu'à une extrémité supérieure. A chacune de ces extrémités, ladite pièce est plus large, formant des pattes en vis-à-vis des éléments contre la face arrière desquels elles viennent en appui et y être fixées. En somme, la partie centrale de chaque pièce présente un rétrécissement, convergent vers le centre instantané des rotations susmentionnées.

De plus, lesdites pièces sont fixées à chacun de ses deux éléments, ainsi que, selon le mode préférentiel de réalisation, en recouvrement de la pièce inférieure.

Pour ce faire, ledit dispositif 1 comprend des moyens de fixation sous la forme d'au moins un couple vis 10 et écrou 11. Ce couple traverse chaque élément pour venir coopérer entre eux. En particulier, chaque vis 10 vient traverser un élément, au niveau d'un orifice 12 ménagé traversant, depuis sa face avant jusqu'à la face arrière, pour venir coopérer par vissage avec l'écrou 11 correspondant.

On notera que chaque orifice 12 peut présenter un bord chanfreiné, de sorte que la tête de ladite vis 10 soit à l'affleurement avec la surface de la face avant de l'élément qu'elle traverse.

De plus, selon le mode préférentiel de réalisation, chaque écrou 11 est inclus au sein de la pièce constituant la liaison 6, 60, 61. Pour ce faire, au moment de la fabrication, lesdits écrous 11 sont directement moulés à l'intérieur de ladite pièce, débouchant du côté de la face venant en appui et au contact de la face arrière de chaque élément, en vis-à-vis des orifices 12.

A ce titre, on notera que ladite face de contact de chaque pièce est prévue incurvée, présentant une concavité, de manière à venir épouser la forme arrondie convexe de la face arrière des éléments contre lesquels elle est fixée.

Comme évoqué précédemment, ledit dispositif 1 peut comprendre deux éléments 2, 3. Il peut aussi comprendre au moins un troisième élément 4 relié supérieurement audit second élément 3 par l'intermédiaire d'une liaison articulée 60 identique à la liaison 6, à savoir qu'elle possède les mêmes caractéristiques, en particulier élastiques, mais qu'elle peut être de forme et de taille différentes. De même, ledit dispositif peut comprendre au moins un quatrième élément 5 relié supérieurement audit troisième élément 4 par l'intermédiaire d'une liaison 61 articulée identique, à savoir qu'elle possède les mêmes caractéristiques, en particulier élastiques, mais qu'elle peut être de forme et de taille différentes.

Par ailleurs, ledit dispositif 1 peut comprendre des moyens d'assujettissement au dos de son porteur. De tels moyens, non représentés, peuvent se présenter sous la forme de sangles, fixées à une extrémité à l'un et/ou l'autre desdits éléments, en fonction de leur nombre. Ces sangles peuvent se rejoindre à leur extrémité opposée respective, formant alors un harnais assurant le maintien en place dudit dispositif 1, mais aussi son maintien en contact et en appui contre le dos de son porteur.

A ce titre, ledit dispositif peut être recouvert au moins en partie d'un revêtement, notamment d'une housse, afin d'améliorer le confort de son port en contact avec la peau. Une telle housse présente alors des dimensions adaptées au dispositif, en particulier au nombre d'éléments qu'il comporte.

Selon une autre réalisation possible, chaque élément peut recevoir un revêtement conformé complémentairement, tel un matelassage sous forme de coussins amovibles, voire une housse.

Selon un mode préférentiel de réalisation, au moins le premier élément 2 peut comprendre une ceinture abdominale, venant entourant le ventre de son porteur. Cette ceinture vient alors se fixer à chaque élément, en particulier du niveau de ses extrémités distales. Ainsi, ladite ceinture vient uniquement soutenir le ventre par l'avant, sans appliquer de force sur l'arrière du dos, au niveau des lombaires. Cette force est transmise à l'élément auquel elle est rattachée.

Selon une caractéristique additionnelle, ledit premier élément 2 peut comprendre inférieurement des moyens 13 de fixation amovible destinés à coopérer avec des moyens complémentaires (non représentés) ménagés au niveau d'un fauteuil roulant, de sorte que ledit dispositif 1 en constitue le dossier.

Ces moyens 13 peuvent être de toute forme et permettent de venir accrocher les moyens complémentaires, notamment par clipsage ou encliquetage. Un système de déverrouillage est alors prévu pour débloquer cet accrochage.

Ainsi, le dispositif 1 selon l'invention offre une coque protectrice modulaire, par le nombre d'éléments 2, 3, 4, 5 qui peuvent la constituer, en vue de s'adapter à la morphologie et les contraintes dorsales du porteur. Elle est aussi ergonomique du fait de sa forme. Principalement, elle autorise les mouvements de son porteur, appliquant une contrainte élastique, assurant le retour en position optimale de maintien du dos dans une position naturelle, mais offrant une résistance permettant d'entretenir le tonus musculaire du dos de son porteur.

## Revendications

1. Dispositif modulaire de protection dorsale (1) adaptée à être porté par un porteur ou formant un dossier articulé personnel à un utilisateur, le dispositif (1) comprenant au moins deux éléments de maintien (2, 3, 4, 5) situés les uns sous les autres et adaptés à recevoir, par leur face avant, le dos du porteur, les éléments de maintien (2, 3, 4, 5)étant reliés entre eux, deux à deux, par leur face arrière, par une liaison articulée (6, 60, 61), chaque liaison articulée (6, 60, 61) assurant le lien entre deux éléments de maintien adjacents (2, 3, 4, 5), chaque liaison articulée (6, 60, 61) comprenant au moins deux degrés de liberté en torsion et en flexion de sorte que chaque élément de maintien (2, 3, 4, 5) et chaque liaison articulée (6, 60, 61) se déplace les uns par rapport aux autres sous l'action des mouvements du porteur ou de l'utilisateur tout en assurant le maintien du dispositif (1) au contact du dos de ce dernier, **caractérisé en ce que** chaque liaison articulée (6, 60, 61) est une liaison élastique, constituée en une unique pièce de jonction en matériau élastique de type élastomère.

2. Dispositif (1) selon la revendication 1, **caractérisée en ce que** chaque pièce de jonction est constituée en polyuréthane.

3. Dispositif (1) selon l'une des revendications 1 et 2, caractérisé en ce chaque liaison articulée (6, 60, 61) est déformable selon un premier axe (7) vertical pour permettre la rotation verticale d'une partie du dos, et selon un second axe (8, 80, 81) horizontale pour permettre le cambrement vers l'arrière et le penchement vers l'avant du porteur.

4. Dispositif (1) selon la revendication 3, **caractérisé en ce que** chaque liaison articulée (6, 60, 61) est déformable selon un troisième axe (9, 90,91) horizontal perpendiculaire au second axe (8, 80,81) correspondant pour permettre au porteur de se pencher vers son côté droit ou son côté gauche.

5. Dispositif (1) selon l'une des revendications 1 à 4, **caractérisé en ce que** chaque élément de maintien (2, 3, 4, 5) est constitué d'une unique lame incurvée en face avant.

6. Dispositif (1) selon la revendication 5, **caractérisé en ce que** la lame incurvée présente, au niveau central, 1 creux orienté vers l'arrière, formée par 1 discontinuité dans la régularité de la courbure de la lame est destinée à recevoir les épineuses de la colonne vertébrale du porteur, le creux étant délimité de part et d'autre des bossages venant en appui de parquet d'autres de la colonne vertébrale.

7. Dispositif (1) selon l'une des revendications 1 à 6, **caractérisé en ce que** chaque liaison articulée (6, 60, 61) constitue des moyens de séparation espacée des éléments de maintien (2, 3, 4, 5) auxquelles ils sont liés de façon à permettre le déplacement relatif de ces derniers sans risque de se toucher.

8. Dispositif (1) Selon l'une des revendications 1 à 7, **caractérisé en ce que** la pièce de jonction à une forme longitudinale et présente deux extrémités larges transversalement formant pattes de fixation, et une partie centrale présentant un rétrécissement.

9. Fauteuil roulant comprenant le dispositif modulaire de protection (1), selon l'une des revendications précédentes, le dispositif modulaire de protection (1) constituant le dossier d'un fauteuil roulant, ledit dispositif (1) comportant un élément de maintien inférieur (2) comprenant inférieurement des moyens (13) de fixation amovible destinés à coopérer avec des moyens complémentaires ménagés au niveau du fauteuil roulant.

## Patentansprüche

1. Modulare Rückenschutzvorrichtung (1), die angepasst ist, um von einem Träger getragen zu werden, oder die eine für einen Benutzer persönliche gelenkige Rückenlehne bildet, wobei die Vorrichtung (1) mindestens zwei Halteelemente (2, 3, 4, 5) umfasst, die sich untereinander befinden und angepasst sind, um mit ihrer Vorderseite den Rücken des Trägers aufzunehmen, wobei die Halteelemente (2, 3, 4, 5) mit ihrer Rückseite paarweise über eine Gelenkverbindung (6, 60, 61) miteinander verbunden sind, wobei jede Gelenkverbindung (6, 60, 61) die Verbindung zwischen zwei benachbarten Halteelementen (2, 3, 4, 5) sichert, wobei jede Gelenkverbindung (6, 60, 61) mindestens zwei Torsions- und Biegefreiheitsgrade umfasst, so dass sich jedes Halteelement (2, 3, 4, 5) und jede Gelenkverbindung (6, 60, 61) relativ zu einander unter der Einwirkung der Bewegungen des Trägers oder des Benutzers bewegen, während das Halten der Vorrichtung (1) in Kontakt mit dem Rücken dieses Letzterren gesichert wird, **dadurch gekennzeichnet, dass** jede Gelenkverbindung (6, 60, 61) eine elastische Verbindung ist, die aus einem einzelnen Verbindungsstück aus elastischem Material der Art Elastomer besteht.

2. Vorrichtung (1) nach Anspruch 1, **dadurch gekennzeichnet, dass** jedes Verbindungsstück aus Polyurethan besteht.

3. Vorrichtung (1) nach einem der Ansprüche 1 und 2, **dadurch gekennzeichnet, dass** jede Gelenkverbindung (6, 60, 61) entlang einer ersten vertikalen Achse (7), um eine vertikale Drehung eines Teils des Rückens zu ermöglichen, und entlang einer zweiten horizontalen Achse (8, 80, 81), um das Rückwärtswölben und das Vorwärtslehnen des Trägers zu ermöglichen, verformbar ist.

4. Vorrichtung (1) nach Anspruch 3, **dadurch gekennzeichnet, dass** jede Gelenkverbindung (6, 60, 61) entlang einer dritten horizontalen Achse (9, 90, 91) senkrecht zur entsprechenden zweiten Achse (8, 80, 81) verformbar ist, um es dem Träger zu ermöglichen, sich zu seiner rechten Seite oder seiner linken Seite zu neigen.

5. Vorrichtung (1) nach einem der Ansprüche 1 bis 4, **dadurch gekennzeichnet, dass** jedes Halteelement (2, 3, 4, 5) aus einer einzigen auf der Vorderseite gekrümmten Klinge besteht.

6. Vorrichtung (1) nach Anspruch 5, **dadurch gekennzeichnet, dass** die gekrümmte Klinge in der Mitte 1 nach hinten gerichtete Vertiefung aufweist, die durch 1 Unterbrechung in der Regelmäßigkeit der Krümmung der Klinge gebildet wird, die dazu bestimmt ist, die Dornfortsätze der Wirbelsäule des Trägers aufzunehmen, wobei die Vertiefung beiderseits der Vorsprünge begrenzt ist, die andere der Wirbelsäule am Boden untestützen.

7. Vorrichtung (1) nach einem der Ansprüche 1 bis 6, **dadurch gekennzeichnet, dass** jede Gelenkverbindung (6, 60, 61) Trennmittel bildet, die von den Halteelementen (2, 3, 4, 5) beabstandet sind, mit denen sie verbunden ist, um die relative Bewegung der letzteren zu ermöglichen, ohne dass die Gefahr besteht, dass sie sich gegenseitig berühren.

8. Vorrichtung (1) nach einem der Ansprüche 1 bis 7, **dadurch gekennzeichnet, dass** das Verbindungsstück eine Längsform hat und zwei breite Enden, die in Querrichtung Befestigungslaschen bilden, und einen Mittelteil, der eine Verengung aufweist, aufweist.

9. Rollstuhl, umfassend die modulare Schutzvorrichtung (1) nach einem der vorhergehenden Ansprüche, wobei die modulare Schutzvorrichtung (1) die Rückenlehne eines Rollstuhls bildet, wobei die besagte Vorrichtung (1) ein unteres Halteelement (2) umfasst, das unten abnehmbare Befestigungsmittel (13) umfasst, die dazu bestimmt sind, mit im Bereich des Rollstuhls vorgesehen ergänzenden Mitteln zusammenzuwirken.

## Claims

1. Modular back protection device (1) adapted to be worn by a wearer or forming a personal articulated backrest for a user, the device (1) comprising at least two holding elements (2, 3, 4, 5) located one below the other and adapted to receive, by their front face, the back of the wearer, the holding elements (2, 3, 4, 5) being pairwise connected to each other by their rear face, by means of an articulated link (6, 60, 61), each articulated link (6, 60, 61) providing the link between two adjacent holding elements (2, 3, 4, 5), each articulated link (6, 60, 61) comprising at least two degrees of freedom in torsion and in bending so that each holding element (2, 3, 4, 5) and each articulated link (6, 60, 61) moves with respect to each other under the action of the movements of the wearer or the user while ensuring maintaining the device (1) in contact with the back of the latter, wherein each articulated link (6, 60, 61) is an elastic link, made up of a single joining part made of an elastic material such as an elastomer.

2. Device (1) according to claim 1, wherein each joining part is made of polyurethane.

3. Device (1) according to one of claims 1 and 2, wherein each articulated link (6, 60, 61) is deformable along a first vertical axis (7) in order to permit the vertical rotation of a portion of the back, and along a second horizontal axis (8, 80, 81) in order to permit the rearward arching and forward leaning of the wearer.

4. Device (1) according to claim 3, wherein each articulated link (6, 60, 61) is deformable along a third horizontal axis (9, 90, 91) perpendicular to the corresponding second axis (8, 80, 81) in order to permit the wearer to lean towards his right or his left.

5. Device (1) according to one of claims 1 to 4, wherein each holding element (2, 3, 4, 5) is formed of a single blade curved on the front face.

6. Device (1) according to claim 5, wherein the curved blade has, at the central level, 1 cavity oriented towards the rear, formed by 1 discontinuity in the regularity of the curvature of the blade intended to receive the outgrowths of the wearer's spine, the cavity being delimited on either side of the bosses supporting as a floor others of the spine.

7. Device (1) according to one of claims 1 to 6, wherein each articulated link (6, 60, 61) constitutes separation means spaced apart from the holding elements (2, 3, 4, 5), which they are connected to, so as to permit the relative displacement of the latter without any risk of touching each other.

8. Device (1) according to one of claims 1 to 7, wherein the joining part has a longitudinal shape and has two wide ends transversely forming fastening lugs, and a central portion having a narrowing.

9. Wheelchair comprising the modular protection device (1) according to one of the preceding claims, the modular protection device (1) forming the backrest of a wheelchair, said device (1) including a lower holding element (2) comprising in its lower portion removable fastening means (13) aimed at cooperating with complementary means arranged at the level of the wheelchair.
